# EUROPEAN PATENT APPLICATION

(11) **EP 1 932 921 A1**
(43) Date of publication of application: **18.06.2008**
(21) Application number: 06126066.7
(22) Date of filing: 13.12.2006
(51) Int. Cl.: C12Q 1/68

(54) **Simple mutant-based differential screening method for compounds modulating a specific target**

(71) Applicant: Novartis AG, 4002 Basel (CH)
(72) Inventor: Helliwell, Stephen, 4143 Dornach (CH); Movva, Rao, 4102 Binningen (CH)
(74) Representative: Bourgarel, Denis Jacques Marie

(57) **Abstract**

Our invention relates to simple rapid and cost-effective screening method for the identification of a target-specific modulator comprising the steps of (a) screening of a library of potential compounds for modulator effects on a non-human organism comprising the target (wild-type), wherein compounds having a modulator effects are identified as potential target-specific modulators, and (b) screening of the potential target-specific modulators identified in step (a) for modulator effects on a mutant of the non-human organism of step (a), wherein said mutant does not comprise the target or wherein said target is deficient in said mutant, wherein a modulator effect in step (b) indicates that said potential target-specific modulators is not target-specific whereas the absence of a modulator effect in step (b) indicates that said potential target-specific modulators is a specific modulator of said target.

## Description

### Field of the invention

The present invention relates to a simple screening method for compounds specifically modulating a target, wherein the use of mutant non-human organisms missing said target allows to quickly select the compounds which are specific from a pool of effective compounds.

### Background of the invention

Classical methods of screening usually require extensive validation of the identified potential modulators of a target in order to demonstrate that said identified potential modulators of a target are specific for said target.
We have developed a simple yeast cell based assay that identifies small molecules that are both active against ScOle1p and specific for ScOle1p. ScOle1p is the yeast equivalent of the HsScd1, the Δ9 fatty acid desaturase that is responsible for the addition of a cis double bond between C9 and C10 of Stearyl-CoA to form oleoyl-CoA. Scd1is currently considered as a target for diabetes and related diseases, because its inhibition results in reduction of diet induced obesity and fatty acid biosynthesis (Jiang et al. 2005).
ScOle1 is basically essential for normal yeast growth, with deletion strains barely able to grow. Addition of the downstream enzymatic product of ScOle1 p, oleic acid, to the growth medium abrogates this growth defect allowing *ole1Δ* strains to grow reasonably well (Stukey et al., 1990). This observation forms the basis of our screen, which comprises 2 assays as described herein.
The skilled person will immediately realise that this approach to identify target-specific compounds among a pool of active ones can be used with any non-human organism wherein a mutant lacking the target is available/can be generated.
Our invention thus provide a simple, flexible and efficient assay circumventing the extensive validation of the identified potential modulators of a target required by the classical screening assays in order to demonstrate that said identified potential modulators of a target are specific for said target.

### Summary of the invention

Our invention hence encompasses a screening method for the identification of a target-specific modulator comprising the steps of (a) screening of a library of potential compounds for modulator effects on a non-human organism comprising the target (wild-type), wherein compounds having a modulator effects are identified as potential target-specific modulators, and (b) screening of the potential target-specific modulators identified in step (a) for modulator effects on a mutant of the non-human organism of step (a), wherein said mutant does not comprise the target or wherein said target is deficient in said mutant, wherein a modulator effect in step (b) indicates that said potential target-specific modulators is not target-specific whereas the absence of a modulator effect in step (b) indicates that said potential target-specific modulators is a specific modulator of said target.
In a preferred embodiment, the non-human organism used is a yeast or a mouse.
In another preferred embodiment the target is a homologue of a human target.
In a particularly preferred embodiment, the target is an essential gene and its absence/deficiency in the mutant non-human organism is specifically compensated by the addition of a heterologuous factor or by an environmental factor, or by alteration of other gene(s) within the same system. The compound to be tested can be an inhibitor or an agonist.
In a most preferred embodiment, the non-human organism is a yeast and the target is an essential gene, so that growth can simply assessed in order to evaluate the specific activity of the inhibitor. For instance, the target can be ScOle1p, the yeast equivalent of the HsScd1, and its absence in the mutant is compensated by the addition of oleic acid to the growth medium.

### Description of the figures

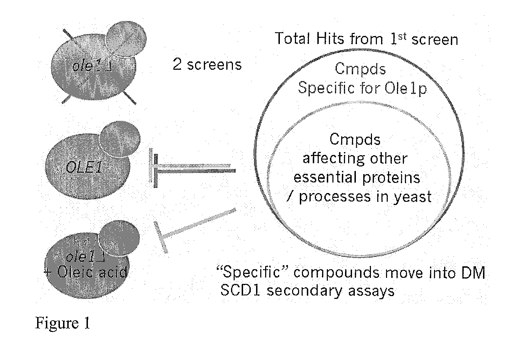

Figure 1: A schematic depicting how the Ole1 screen would be performed. Wild type yeast cells would be assayed for growth inhibition using compound libraries. The total hits are represented by the contents of both circles on the right hand side. All of these hits are then rescreened against a *ole1*Δ yeast strain grown upon media containing oleic acid. Compounds that no longer inhibit are considered specific for Ole 1p. Compounds that are still anti-proliferative are considered to be unspecific, and are no longer pursued.

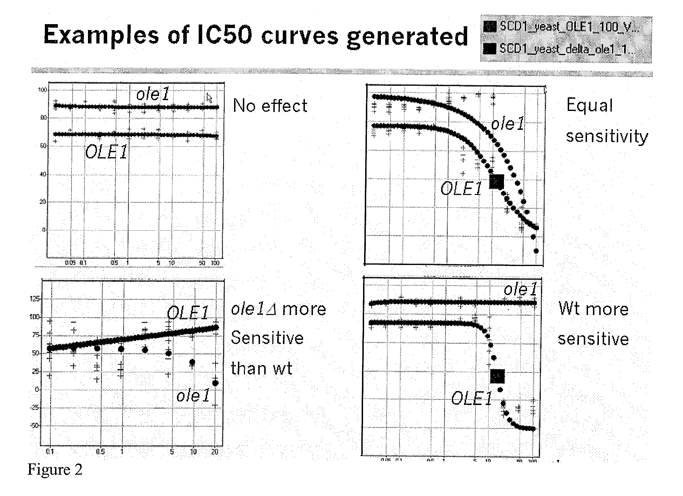

Figure2: Examples of compound behaviour in this assay. Shown are 4 plots of the differential proliferation assay carried out using serial dilutions of compound, and either the wild type strain (*OLE1*) or the *ole1Δ* strain grown in oleic acid media (*ole1*). The lower right panel illustrates the shape of plots one would expect from a positive outcome i.e. the wild type strain is clearly affected at a particular concetration, whilst the *ole1Δ* strain is unaffected thoughout the concentration ranges utilised.

### Detailed description of the invention

We have developed a simple yeast cell based assay that identifies small molecules that are both active against ScOle1p and specific for ScOle1p. ScOle1p is the yeast equivalent of the HsScd1, the Δ9 fatty acid desaturase that is responsible for the addition of a cis double bond between C9 and C10 of Stearyl-CoA to form oleoyl-CoA. Scd1 is currently considered as a target for diabetes and related diseases, because its inhibition results in reduction of diet induced obesity and fatty acid biosynthesis (Jiang et al. 2005).
ScOle1 is basically essential for normal yeast growth, with deletion strains barely able to grow. Addition of the downstream enzymatic product of ScOle1 p, oleic acid, to the growth medium abrogates this growth defect allowing *ole1Δ* strains to grow reasonably well (Stukey et al., 1990).
This observation forms the basis of our screen, which comprises 2 assays. In a primary assay small molecules would be screened against wild type yeast for molecules which reduce or stop growth - these molecules would therefore affect (amongst other things) proteins that are essential for growth under the screen conditions. All the "hits" from the primary assay would then be re-screened in a secondary assay against an *ole1*Δ strain grown in the presence of oleic acid, such that small molecules that directly and specifically target ScOle1 p and no other essential processes would not affect the growth of the *ole1Δ* strain grown in the presence of oleic acid. These molecules would be considered specific for the ScOle1p - and represent molecules to be followed up. Small molecule "hits" that do / do not affect ScOle1p but affect some other essential protein will still score as growth inhibitory, and would no longer be pursued.

The skilled person will immediately realise that this approach can be expanded to include any essential yeast gene that has (i) a human homologue (that someone wishes to screen), and (ii) some way of bypassing the need for the gene in question, either as above (enzyme product addition), or by utilising downstream mutations that overcome the loss of the gene in question. Preferred examples of such genes can be found in Table 1.

**Table 1**

| **Yeast Systematic Gene** | **Yeast Common Name** | **Hs Subject id** | **Hs Subj Locus** | **% identity** | **Genetic Rescue ?** |
|---|---|---|---|---|---|
| YBR236C | ABD1 | NP_003790 | RNMT | 33.64 | Dosage |
| | | | | | Rescue |
| YNR016C | ACC1 | NP_001084 | ACACB | 46.91 | |
| YKL192C | ACP1 | NP_004994 | NDUFAB1 | 45.24 | |
| YLR153C | ACS2 | NP_061147 | ACSS2 | 45.4 | |
| YFL039C | ACT1 | NP_001605 | ACTG1 | 89.07 | Dosage |
| | | | | | Rescue |
| YLR359W | ADE13 | NP_000017 | ADSL | 64.39 | Synthetic |
| | | | | | Rescue |
| YLR397C | AFG2 | NP_660208 | SPATA5 | 48.28 | |
| YOR335C | ALA1 | NP_001596 | AARS | 51.03 | |
| YBR110W | ALG1 | NP_061982 | ALG1 | 35.78 | Dosage |
| | | | | | Rescue |
| YGL047W | ALG13 | NP_060936 | GLT28D1 | 28.35 | |
| YBR070C | ALG14 | NP_659425 | ALG14 | 35.38 | |
| YGL065C | ALG2 | NP_149078 | ALG2 | 37.73 | Dosage |
| | | | | | Rescue |
| YBR243C | ALG7 | NP_001373 | DPAGT1 | 42.18 | |
| YPR180W | AOS1 | NP_005491 | SAE1 | 34.02 | |
| YNL172W | APC1 | NP_073153 | ANAPC1 | 25.59 | |
| YDL008W | APC11 | NP_001002245 | ANAPC11 | 44.12 | |
| YLR127C | APC2 | NP_037498 | ANAPC2 | 23.15 | Dosage |
| | | | | | Rescue |
| YIL062C | ARC15 | NP_112240 | ARPC5L | 28.39 | |
| YKL013C | ARC19 | NP_005709 | ARPC4 | 68.64 | |
| YNR035C | ARC35 | NP_690601 | ARPC2 | 36.34 | Dosage |
| | | | | | Rescue |
| YBR234C | ARC40 | NP_006400 | ARPC1A | 37.2 | |
| YDR376W | ARH1 | NP_077728 | FDXR | 33.74 | |
| YDL029W | ARP2 | NP_005713 | ACTR2 | 67.89 | Synthetic |
| | | | | | Rescue |
| YJR065C | ARP3 | NP_065178 | ACTR3B | 57.46 | Dosage |
| | | | | | Rescue |
| YJL081C | ARP4 | NP_057272 | ACTL6B | 29.2 | |
| YPR034W | ARP7 | NP_001017421 | FKSG30 | 26.82 | Dosage |
| | | | | | Rescue |
| YMR301C | ATM1 | NP_004290 | ABCB7 | 49.75 | Dosage |
| | | | | | Rescue |
| YDL004W | ATP16 | NP_001001975 | ATP5D | 35.64 | |
| YHR040W | BCD1 | NP_060423 | C1orf181 | 29.38 | |
| YNL039W | BDP1 | NP_060899 | BDP1 | 30.62 | Dosage |
| | | | | | Rescue |
| YPR176C | BET2 | NP_004573 | RABGGTB | 52.2 | |
| YKR068C | BET3 | NP_055223 | TRAPPC3 | 56.07 | Dosage |
| | | | | | Rescue |
| YJL031C | BET4 | NP_004572 | RABGGTA | 31.62 | |
| YML077W | BET5 | NP_067033 | TRAPPC1 | 32.79 | Dosage |
| | | | | | Rescue |
| YDR299W | BFR2 | NP_036270 | AATF | 26.72 | |
| YPL217C | BMS1 | NP_055568 | BMS1L | 44.55 | |
| YDL141W | BPL1 | NP_000402 | HLCS | 27.94 | |
| YGR246C | BRF1 | NP_001510 | BRF1 | 42.72 | Dosage |
| | | | | | Rescue |
| YBL097W | BRN1 | NP_056156 | BRRN1 | 20.65 | Dosage |
| | | | | | Rescue |
| YER172C | BRR2 | NP_054733 | ASCC3L1 | 39.54 | Synthetic |
| | | | | | Rescue |
| YOL077C | BRX1 | NP_060791 | BXDC2 | 53.12 | Dosage |
| | | | | | Rescue |
| YER159C | BUR6 | NP_006433 | DRAP1 | 45.57 | Dosage |
| | | | | | Rescue |
| YFL029C | CAK1 | NP_001789 | CDK2 | 26.27 | Synthetic |
| | | | | | Rescue |
| YLR175W | CBF5 | NP_001354 | DKC1 | 71.61 | Dosage |
| | | | | | Rescue |
| YNL161W | CBK1 | NP_055815 | STK38L | 47.93 | Synthetic |
| | | | | | Rescue |
| YER168C | CCA1 | NP_057084 | TRNT1 | 32.19 | |
| YPR025C | CCL1 | NP_001230 | CCNH | 25.56 | |
| YIL142W | CCT2 | NP_006422 | CCT2 | 65.71 | Dosage |
| | | | | | Rescue |
| YJL014W | CCT3 | NP_005989 | CCT3 | 59.7 | Dosage |
| | | | | | Rescue |
| YDL143W | CCT4 | NP_006421 | CCT4 | 59.42 | |
| YJR064W | CCT5 | NP_036205 | CCT5 | 59.71 | |
| YDR188W | CCT6 | NP_001753 | CCT6A | 56.75 | |
| YJL111W | CCT7 | NP_006420 | CCT7 | 63.08 | |
| YJL008C | CCT8 | NP_006576 | CCT8 | 45.72 | |
| YDR182W | CDC1 | NP_075563 | MPPE1 | 23.47 | Synthetic |
| | | | | | Rescue |
| YJR076C | CDC11 | NP_002679 | SEP5 | 41.4 | Dosage |
| | | | | | Rescue |
| YHR107C | CDC12 | NP_001779 | SEP7 | 37.44 | Dosage |
| | | | | | Rescue |
| YLR215C | CDC123 | NP_006014 | C10orf7 | 35.06 | |
| YFR028C | CDC14 | NP_201570 | CDC14A | 37.5 | Synthetic |
| | | | | | Rescue |
| YAR019C | CDC15 | NP_006273 | STK4 | 40.46 | Synthetic |
| | | | | | Rescue |
| YKL022C | CDC16 | NP_003894 | CDC16 | 32.65 | Synthetic |
| | | | | | Rescue |
| YAL038W | CDC19 | NP_002645 | PKM2 | 52.04 | Dosage |
| | | | | | Rescue |
| YDL102W | CDC2 | NP_002682 | POLD1 | 49.05 | Synthetic |
| | | | | | Rescue |
| YGL116W | CDC20 | NP_001246 | CDC20 | 34.1 | Synthetic |
| | | | | | Rescue |
| YOR074C | CDC21 | NP_001062 | TYMS | 62.37 | Dosage |
| | | | | | Rescue |
| YHR166C | CDC23 | NP_004652 | CDC23 | 30.85 | Synthetic |
| | | | | | Rescue |
| YAL041 W | CDC24 | NP_006104 | VAV3 | 21.9 | Dosage |
| | | | | | Rescue |
| YLR310C | CDC25 | NP_005624 | SOS1 | 28.98 | Synthetic |
| | | | | | Rescue |
| YBL084C | CDC27 | NP_001247 | CDC27 | 39.68 | Synthetic |
| | | | | | Rescue |
| YBR160W | CDC28 | NP_001249 | CDK3 | 64.93 | Dosage |
| | | | | | Rescue |
| YLR314C | CDC3 | NP_001779 | SEP7 | 42.89 | |
| YOR257W | CDC31 | NP_004356 | CETN3 | 61.81 | Dosage |
| | | | | | Rescue |
| YOL139C | CDC33 | NP_001959 | EIF4E | 37.02 | Synthetic |
| | | | | | Rescue |
| YDR054C | CDC34 | NP_060281 | UBE2R2 | 35.56 | Dosage |
| | | | | | Rescue |
| YDL165W | CDC36 | NP_055330 | CNOT2 | 28.65 | Dosage |
| | | | | | Rescue |
| YCR093W | CDC39 | NP_057368 | CNOT1 | 28.26 | Synthetic |
| | | | | | Rescue |
| YFL009W | CDC4 | NP_060785 | FBXW7 | 29.39 | Dosage |
| | | | | | Rescue |
| YLR229C | CDC42 | NP_001782 | CDC42 | 80.1 | Dosage |
| | | | | | Rescue |
| YGL155W | CDC43 | NP_005014 | PGGT1B | 28.45 | Synthetic |
| | | | | | Rescue |
| YLR103C | CDC45 | NP_003495 | CDC45L | 23.48 | Synthetic |
| | | | | | Rescue |
| YLR274W | CDC46 | NP_006730 | MCM5 | 46.41 | Synthetic |
| | | | | | Rescue |
| YBR202W | CDC47 | NP_005907 | MCM7 | 45.76 | Dosage |
| | | | | | Rescue |
| YDL126C | CDC48 | NP_009057 | VCP | 70.23 | |
| YMR001C | CDC5 | NP_006613 | PLK2 | 33.08 | Dosage |
| | | | | | Rescue |
| YDL132W | CDC53 | NP_003583 | CUL1 | 31.8 | Dosage |
| | | | | | Rescue |
| YPR019W | CDC54 | NP_877423 | MCM4 | 47.4 | |
| YJL194W | CDC6 | NP_001245 | CDC6 | 25.71 | Synthetic |
| | | | | | Rescue |
| YPL160W | CDC60 | NP_064502 | LARS | 46.14 | |
| YDL017W | CDC7 | NP_003494 | CDC7 | 37.99 | Dosage |
| | | | | | Rescue |
| YJR057W | CDC8 | NP_036277 | DTYMK | 50.6 | Dosage |
| | | | | | Rescue |
| YDL164C | CDC9 | NP_000225 | LIG1 | 40.9 | |
| YBR029C | CDS1 | NP_001254 | CDS1 | 40.15 | Synthetic |
| | | | | | Rescue |
| YMR213W | CEF1 | NP_001244 | CDC5L | 50.79 | Synthetic |
| | | | | | Rescue |
| YGL130W | CEG1 | NP_003791 | RNGTT | 28.77 | Dosage |
| | | | | | Rescue |
| YIL003W | CFD1 | NP_036357 | NUBP2 | 54.62 | |
| YDR301W | CFT1 | NP_037423 | CPSF1 | 25.24 | |
| YLR115W | CFT2 | NP_059133 | CPSF2 | 26.22 | |
| YBR135W | CKS1 | NP_001818 | CKS2 | 53.25 | |
| YLR117C | CLF1 | NP_057736 | CRNKL1 | 37.65 | Dosage |
| | | | | | Rescue |
| YOR250C | CLP1 | NP_006822 | HEAB | 24.14 | |
| YBR109C | CMD1 | NP_001734 | CALM2 | 59.86 | Dosage |
| | | | | | Rescue |
| YBR155W | CNS1 | NP_004614 | TTC4 | 27.99 | Dosage |
| | | | | | Rescue |
| YLL050C | COF1 | NP_005498 | CFL1 | 37.76 | Dosage |
| | | | | | Rescue |
| YER157W | COG3 | NP_113619 | COG3 | 22.38 | Dosage |
| | | | | | Rescue |
| YPR105C | COG4 | NP_056201 | COG4 | 19.33 | Dosage |
| | | | | | Rescue |
| YDL145C | COP1 | NP_004362 | COPA | 43.74 | Dosage |
| | | | | | Rescue |
| YLL018C-A | COX19 | NP_001026788 | LOC90639 | 40.26 | |
| YGR218W | CRM1 | NP_003391 | XP01 | 46.99 | Dosage |
| | | | | | Rescue |
| YGL238W | CSE1 | NP_001307 | CSE1L | 36.83 | Dosage |
| | | | | | Rescue |
| YKL049C | CSE4 | NP_001005464 | H3 | 67.01 | Dosage |
| | | | | | Rescue |
| YNL232W | CSL4 | NP_057130 | EXOSC1 | 47.96 | |
| YCR054C | CTR86 | NP_037368 | ATXN10 | 36.63 | |
| YMR240C | CUS1 | NP_006833 | SF3B2 | 30.17 | |
| YGR278W | CWC22 | NP_065994 | KIAA1604 | 27.8 | |
| YLR323C | CWC24 | NP_008909 | RNF113A | 42.86 | |
| YJL005W | CYR1 | NP_055835 | PHLPPL | 23.8 | Synthetic |
| | | | | | Rescue |
| YDL031 W | DBP10 | NP_076977 | DDX54 | 37.5 | |
| YNL112W | DBP2 | NP_004387 | DDX5 | 58.56 | |
| YOR046C | DBP5 | NP_060802 | DDX19A | 47.41 | Synthetic |
| | | | | | Rescue |
| YNR038W | DBP6 | NP_778236 | DDX51 | 32.04 | Dosage |
| | | | | | Rescue |
| YLR276C | DBP9 | NP_061955 | DDX56 | 42.21 | |
| YNL118C | DCP2 | NP_689837 | DCP2 | 37.12 | Dosage |
| | | | | | Rescue |
| YOR204W | DED1 | NP_004651 | DDX3Y | 58.96 | Synthetic |
| | | | | | Rescue |
| YHR019C | DED81 | NP_004530 | NARS | 51.35 | |
| YOR236W | DFR1 | NP_000782 | DHFR | 30.77 | |
| YKL078W | DHR2 | NP_004932 | DHX8 | 38.96 | |
| YPR082C | DIB1 | NP_006692 | TXNL4A | 65.94 | |
| YPL266W | DIM1 | NP_055288 | HSA9761 | 59.86 | |
| YLR129W | DIP2 | NP_006775 | WDR3 | 36.9 | |
| YOL021C | DIS3 | NP_055768 | KIAA1008 | 43.06 | |
| YHR164C | DNA2 | XP_166103 | DNA2L | 32.44 | Synthetic |
| | | | | | Rescue |
| YDR141C | DOP1 | NP_055833 | KIAA1117 | 26.37 | |
| YPR175W | DPB2 | NP_002683 | POLE2 | 26.41 | Dosage |
| | | | | | Rescue |
| YPR183W | DPM1 | NP_003850 | DPM1 | 32.77 | Dosage |
| | | | | | Rescue |
| YLL018C | DPS1 | NP_001340 | DARS | 57.26 | |
| YLL008W | DRS1 | NP_060365 | DDX27 | 43.87 | |
| YBR252W | DUT1 | NP_001020420 | DUT | 55.07 | |
| YHR068W | DYS1 | NP_001921 | DHPS | 57.69 | |
| YKL172W | EBP2 | NP_006815 | EBNA1BP2 | 38.73 | |
| YMR128W | ECM16 | NP_116045 | DHX37 | 54.11 | |
| YFR027W | ECO1 | NP_443143 | ESCO1 | 21.92 | Dosage |
| | | | | | Rescue |
| YAL003W | EFB1 | NP_066944 | EEF1B2 | 46.9 | Dosage |
| | | | | | Rescue |
| YLR186W | EMG1 | NP_006322 | C2F | 51.24 | Dosage |
| | | | | | Rescue |
| YBR247C | ENP1 | NP_004044 | BYSL | 33.89 | |
| YGR145W | ENP2 | NP_079170 | NOL10 | 39.87 | |
| YFL024C | EPL1 | NP_079485 | EPC1 | 24.07 | |
| YMR049C | ERB1 | NP_056016 | BOP1 | 40.92 | |
| YBL040C | ERD2 | NP_006792 | KDELR1 | 50.46 | Dosage |
| | | | | | Rescue |
| YGR175C | ERG1 | NP_003120 | SQLE | 36.67 | |
| YPL028W | ERG10 | NP_000010 | ACAT1 | 47.49 | |
| YHR007C | ERG11 | NP_000777 | CYP51A1 | 36.68 | Synthetic |
| | | | | | Rescue |
| YMR208W | ERG12 | NP_000422 | MVK | 32.2 | |
| YML126C | ERG13 | NP_002121 | HMGCS1 | 48.58 | Synthetic |
| | | | | | Rescue |
| YJL167W | ERG20 | NP_001995 | FDPS | 45.06 | |
| YGR060W | ERG25 | NP_006736 | SC4MOL | 37.46 | Synthetic |
| | | | | | Rescue |
| YGL001C | ERG26 | NP_057006 | NSDHL | 38.46 | |
| YLR100W | ERG27 | NP_057455 | HSD17B7 | 22.54 | |
| YHR072W | ERG7 | NP_002331 | LSS | 41.03 | |
| YHR190W | ERG9 | NP_004453 | FDFT1 | 48.24 | |
| YML130C | ER01 | NP_063944 | ERO1LB | 34.87 | Dosage |
| | | | | | Rescue |
| YGR029W | ERV1 | NP_005253 | GFER | 46.85 | |
| YOR244W | ESA1 | NP_874368 | HTATIP | 46.22 | |
| YDR365C | ESF1 | NP_057733 | C20orf6 | 31.09 | |
| YNR054C | ESF2 | NP_037507 | ABT1 | 28.19 | |
| YGR098C | ESP1 | NP_036423 | ESPL1 | 27.85 | Synthetic |
| | | | | | Rescue |
| YJR017C | ESS1 | NP_006212 | PIN1 | 46.34 | Synthetic |
| | | | | | Rescue |
| YDL045C | FAD1 | NP_958800 | FLAD1 | 31.36 | |
| YDR021W | FAL1 | NP_055555 | DDX48 | 62.73 | |
| YDL166C | FAP7 | NP_057367 | TAF9 | 40.48 | |
| YMR277W | FCP1 | NP_430255 | CTDP1 | 33.6 | Dosage |
| | | | | | Rescue |
| YPR104C | FHL1 | NP_060886 | FOXJ2 | 42.86 | Dosage |
| | | | | | Rescue |
| YJR093C | FIP1 | NP_112179 | FIP1L1 | 48.28 | |
| YDR236C | FMN1 | NP_060809 | RFK | 36.25 | |
| YGR267C | FOL2 | NP_001019195 | GCH1 | 58.77 | |
| YMR113W | FOL3 | NP_004948 | FPGS | 28.86 | |
| YDR373W | FRQ1 | NP_055101 | FREQ | 60 | Dosage |
| | | | | | Rescue |
| YLR060W | FRS1 | NP_005678 | FARSLB | 45.48 | |
| YFL022C | FRS2 | NP_004452 | FARSLA | 49.79 | |
| YLR088W | GAA1 | NP_003792 | GPAA1 | 28.1 | |
| YLR459W | GAB1 | NP_536724 | CDC91L1 | 27.7 | Dosage |
| | | | | | Rescue |
| YOR260W | GCD1 | NP_065098 | EIF2B3 | 22.73 | Synthetic |
| | | | | | Rescue |
| YNL062C | GCD10 | NP_057023 | CGI-09 | 23.76 | Dosage |
| | | | | | Rescue |
| YER025W | GCD11 | NP_001406 | EIF2S3 | 73.87 | |
| YJL125C | GCD14 | NP_689520 | C14orf172 | 40.3 | Dosage |
| | | | | | Rescue |
| YGR083C | GCD2 | NP_751945 | EIF2B4 | 36.29 | Dosage |
| | | | | | Rescue |
| YDR211W | GCD6 | NP_003898 | EIF2B5 | 28.55 | |
| YLR291C | GCD7 | NP_055054 | EIF2B2 | 32.15 | |
| YER136W | GDI1 | NP_001485 | GDI2 | 56.35 | |
| YKL104C | GFA1 | NP_005101 | GFPT2 | 55.54 | |
| YER133W | GLC7 | NP_002701 | PPP1CC | 85.76 | Synthetic |
| | | | | | Rescue |
| YPR035W | GLN1 | NP_001028216 | GLUL | 56.07 | |
| YOR168W | GLN4 | NP_005042 | QARS | 39.28 | |
| YFL017C | GNA1 | NP_932332 | GNPNAT1 | 32.88 | |
| YGR216C | GPI1 | NP_004195 | PIGQ | 32.69 | |
| YGL142C | GPI10 | NP_004846 | PIGB | 26.7 | |
| YMR281W | GPI12 | NP_004269 | PIGL | 32.08 | |
| YLL031C | GPI13 | NP_116023 | PIGO | 32.95 | Dosage |
| | | | | | Rescue |
| YJR013W | GPI14 | NP_660150 | PIGM | 37.31 | |
| YHR188C | GPI16 | NP_057021 | PIGT | 30.02 | |
| YDR434W | GPI17 | NP_149975 | PIGS | 23.1 | |
| YDR331 W | GPI8 | NP_005473 | PIGK | 60.59 | |
| YKL152C | GPM1 | NP_000281 | PGAM2 | 52.23 | |
| YBR121C | GRS1 | NP_002038 | GARS | 46.4 | |
| YDR454C | GUK1 | NP_000849 | GUK1 | 51.35 | Dosage |
| | | | | | Rescue |
| YGL245W | GUS1 | NP_004437 | EPRS | 45.33 | |
| YJL091C | GWT1 | NP_848612 | PIGW | 30.19 | |
| YMR290C | HAS1 | NP_006764 | DDX18 | 59.76 | |
| YJL033W | HCA4 | NP_004389 | DDX10 | 46.14 | Synthetic |
| | | | | | Rescue |
| YDR232W | HEM1 | NP_000023 | ALAS2 | 43.66 | |
| YDR047W | HEM12 | NP_000365 | UROD | 52.11 | Synthetic |
| | | | | | Rescue |
| YDR044W | HEM13 | NP_000088 | CPOX | 52.52 | |
| YOR176W | HEM15 | NP_000131 | FECH | 51.3 | |
| YGL040C | HEM2 | NP_000022 | ALAD | 53.35 | |
| YDL205C | HEM3 | NP_001019553 | HMBS | 39.23 | |
| YGR191W | HIP1 | NP_003036 | SLC7A1 | 22 | |
| YOL123W | HRP1 | NP_002433 | MS11 | 46.59 | |
| YPL204W | HRR25 | NP_001885 | CSNK1 E | 65.77 | |
| YOL133W | HRT1 | NP_055063 | RBX1 | 66.29 | Dosage |
| | | | | | Rescue |
| YGL073W | HSF1 | NP_005517 | HSF1 | 30.6 | Dosage |
| | | | | | Rescue |
| YMR288W | HSH155 | NP_036565 | SF3B1 | 50.98 | |
| YOR319W | HSH49 | NP_005841 | SF3B4 | 37.76 | |
| YOR020C | HSP10 | NP_002148 | HSPE1 | 46.81 | |
| YLR259C | HSP60 | NP_002147 | HSPD1 | 56.98 | |
| YDR224C | HTB1 | NP_003510 | HST1H2BL | 80 | |
| YPR033C | HTS1 | NP_002100 | HARS | 50.1 | |
| YKL189W | HYM1 | NP_057373 | CAB39 | 33.91 | Synthetic |
| | | | | | Rescue |
| YEL034W | HYP2 | NP_001961 | EIFSA | 64.19 | Dosage |
| | | | | | Rescue |
| YJR006W | HYS2 | NP_006221 | POLD2 | 29.38 | Synthetic |
| | | | | | Rescue |
| YPL117C | IDI1 | NP_004499 | IDI1 | 48.68 | |
| YBL076C | ILS1 | NP_038203 | IARS | 53.98 | |
| YHR148W | IMP3 | NP_060755 | IMP3 | 53.85 | |
| YNL075W | IMP4 | NP_219484 | IMP4 | 51.54 | |
| YGL150C | INO80 | NP_060023 | INOC1 | 50.88 | |
| YHR085W | IPI1 | NP_060216 | TEX10 | 30.37 | |
| YNL182C | IPI3 | NP_077005 | WDR18 | 21.74 | |
| YPL209C | IPL1 | NP_940839 | STK6 | 43.6 | Synthetic |
| | | | | | Rescue |
| YBR011C | IPP1 | NP_066952 | PPA1 | 52.98 | |
| YPL242C | IQG1 | NP_839943 | IQGAP3 | 36.73 | Dosage |
| | | | | | Rescue |
| YBR140C | IRA1 | NP_000258 | NF1 | 22.02 | Dosage |
| | | | | | Rescue |
| YIL026C | IRR1 | NP_005853 | STAG1 | 26.01 | Dosage |
| | | | | | Rescue |
| YPR133C | IWS1 | NP_060439 | FLJ10006 | 28.19 | |
| YGL018C | JAC1 | NP_741999 | HSC20 | 28.67 | Dosage |
| | | | | | Rescue |
| YKR038C | KAE1 | NP_060277 | OSGEP | 61.41 | |
| YLR347C | KAP95 | NP_002256 | KPNB1 | 33.45 | |
| YJL034W | KAR2 | NP_005338 | HSPA5 | 68.57 | Dosage |
| | | | | | Rescue |
| YDL108W | KIN28 | NP_001790 | CDK7 | 48.3 | Dosage |
| | | | | | Rescue |
| YHR186C | KOG1 | NP_065812 | raptor | 55.61 | Dosage |
| | | | | | Rescue |
| YNL132W | KRE33 | NP_078938 | FLJ10774 | 55.76 | |
| YOR336W | KRE5 | NP_001020948 | UGCGL1 | 26.56 | Dosage |
| | | | | | Rescue |
| YCL059C | KRR1 | NP_008974 | HRB2 | 64.16 | Dosage |
| | | | | | Rescue |
| YDR037W | KRS1 | NP_005539 | KARS | 55.69 | |
| YKR063C | LAS1 | NP_112483 | LAS1L | 34.39 | |
| YOR181W | LAS17 | NP_000368 | WAS | 34.95 | Synthetic |
| | | | | | Rescue |
| YMR296C | LCB1 | NP_006406 | SPTLC1 | 33.83 | Synthetic |
| | | | | | Rescue |
| YDR062W | LCB2 | NP_004854 | SPTLC2 | 47.95 | |
| YGL099W | LSG1 | NP_060855 | FLJ11301 | 58.64 | Dosage |
| | | | | | Rescue |
| YBL026W | LSM2 | NP_067000 | LSM2 | 63.16 | |
| YLR438C-A | LSM3 | NP_055278 | LSM3 | 41.18 | |
| YER112W | LSM4 | NP_036453 | LSM4 | 40.86 | |
| YER146W | LSM5 | NP_036454 | LSM5 | 51.22 | Synthetic |
| | | | | | Rescue |
| YNL006W | LST8 | NP_071767 | GBL | 45.85 | Dosage |
| | | | | | Rescue |
| YDL087C | LUC7 | NP_958815 | LUC7L | 34.19 | |
| YKL021C | MAK11 | NP_060376 | PAK11P1 | 25.59 | |
| YAL025C | MAK16 | NP_115898 | RBM13 | 56.5 | |
| YDR060W | MAK21 | NP_005751 | CEBPZ | 32.05 | |
| YBR142W | MAK5 | NP_065147 | DDX24 | 32.93 | |
| YLR163C | MAS1 | NP_004270 | PMPCB | 44.01 | Dosage |
| | | | | | Rescue |
| YHR024C | MAS2 | NP_055975 | PMPCA | 37.53 | |
| YKL165C | MCD4 | NP_789744 | PIGN | 37.42 | Dosage |
| | | | | | Rescue |
| YMR043W | MCM1 | NP_003122 | SRF | 67.5 | Synthetic |
| | | | | | Rescue |
| YIL150C | MCM10 | NP_877428 | MCM10 | 22.35 | Synthetic |
| | | | | | Rescue |
| YBL023C | MCM2 | NP_004517 | MCM2 | 47.78 | Synthetic |
| | | | | | Rescue |
| YEL032W | MCM3 | NP_002379 | MCM3 | 51.77 | Synthetic |
| | | | | | Rescue |
| YGL201C | MCM6 | NP_005906 | MCM6 | 47.25 | |
| YLR106C | MDN1 | NP_055426 | MDN1 | 40.51 | |
| YBR136W | MEC1 | NP_001175 | ATR | 24.28 | Synthetic |
| | | | | | Rescue |
| YHR058C | MED6 | NP_005457 | MED6 | 28.14 | Synthetic |
| | | | | | Rescue |
| YOL135C | MED7 | NP_004261 | CRSP9 | 34 | |
| YGR264C | MES1 | NP_004981 | MARS | 51.39 | |
| YIL046W | MET30 | NP_060785 | FBXW7 | 31.22 | Dosage |
| | | | | | Rescue |
| YPL169C | MEX67 | NP_006353 | NXF1 | 21.99 | Dosage |
| | | | | | Rescue |
| YOR232W | MGE1 | NP_079472 | GRPEL1 | 39.49 | |
| YKL195W | MIA40 | NP_653237 | CHCHD4 | 55.29 | |
| YIL051C | MMF1 | NP_005827 | HRSP12 | 36.36 | Dosage |
| | | | | | Rescue |
| YGL068W | MNP1 | NP_002940 | MRPL12 | 38.89 | |
| YIL106W | MOB1 | NP_060691 | MOBK1B | 50.47 | Dosage |
| | | | | | Rescue |
| YFL034C-B | MOB2 | NP_775739 | MOBKL1A | 41.44 | Synthetic |
| | | | | | Rescue |
| YPL082C | MOT1 | NP_003963 | BTAF1 | 36.37 | Synthetic |
| | | | | | Rescue |
| YKL059C | MPE1 | NP_061173 | RBBP6 | 30.7 | |
| YJR002W | MPP10 | NP_005782 | MPHOSPH10 | 30.99 | |
| YDL028C | MPS1 | NP_003309 | TTK | 37.76 | Dosage |
| | | | | | Rescue |
| YPR112C | MRD1 | NP_057280 | RBM19 | 31.19 | |
| YHR005C-A | MRS11 | NP_036588 | TIMM10 | 41.27 | |
| YOR370C | MRS6 | NP_001485 | GDI2 | 26.7 | Dosage |
| | | | | | Rescue |
| YLR116W | MSL5 | NP_973727 | SF1 | 43.42 | |
| YDR208W | MSS4 | NP_003548 | PIP5K1A | 36.9 | Dosage |
| | | | | | Rescue |
| YOR160W | MTR10 | NP_036602 | TNPO3 | 24.4 | |
| YJL050W | MTR4 | NP_056175 | SKIV2L2 | 55.14 | |
| YNR043W | MVD1 | NP_002452 | MVD | 47.57 | |
| YHR023W | MYO1 | NP_005955 | MYH10 | 31.11 | Dosage |
| | | | | | Rescue |
| YOR326W | MYO2 | XP_371116 | MY05B | 40.86 | Dosage |
| | | | | | Rescue |
| YGL122C | NAB2 | NP_997543 | FLJ11806 | 23.64 | Dosage |
| | | | | | Rescue |
| YNL240C | NAR1 | NP_071938 | NARFL | 30.52 | |
| YGL091C | NBP35 | NP_002475 | NUBP1 | 53.82 | |
| YDR397C | NCB2 | NP_001929 | DR1 | 36.7 | |
| YHR042W | NCP1 | NP_000932 | POR | 33.8 | |
| YIL048W | NEO1 | NP_940933 | ATP9B | 50.1 | |
| YCL017C | NFS1 | NP_066923 | NFS1 | 67.33 | |
| YDL208W | NHP2 | NP_060308 | NOLA2 | 48.48 | |
| YFR002W | NIC96 | NP_055484 | NUP93 | 24.83 | Dosage |
| | | | | | Rescue |
| YMR309C | NIP1 | NP_003743 | EIF3S8 | 28.25 | Synthetic |
| | | | | | Rescue |
| YPL211W | NIP7 | NP_057185 | CGI-37 | 56.98 | |
| YHR170W | NMD3 | NP_057022 | NMD3 | 45.14 | |
| YLR195C | NMT1 | NP_004799 | NMT2 | 45.01 | Dosage |
| | | | | | Rescue |
| YOR056C | NOB1 | NP_054781 | NOB1P | 30.77 | |
| YOR206W | NOC2 | NP_056473 | NOC2L | 30.39 | |
| YLR002C | NOC3 | NP_071896 | NOC3L | 30.48 | |
| YPR144C | NOC4 | NP_076983 | NOC4L | 30.51 | |
| YPL093W | NOG1 | NP_036473 | GTPBP4 | 46.2 | |
| YNR053C | NOG2 | NP_037417 | GNL2 | 55.53 | |
| YDL014W | NOP1 | NP_001427 | FBL | 71.06 | |
| YHR072W-A | NOP10 | NP_061118 | NOLA3 | 60.34 | |
| YDL148C | NOP14 | NP_003694 | C4orf9 | 26.59 | |
| YNL110C | NOP15 | NP_115766 | MKI67IP | 33.12 | |
| YNL061W | NOP2 | NP_001028886 | NOL1 | 61.9 | |
| YPL043W | NOP4 | NP_060547 | RBM28 | 26.2 | |
| YOR310C | NOP58 | NP_057018 | NOP5 | 50.8 | |
| YGR103W | NOP7 | NP_055118 | PES1 | 43.67 | |
| YJR072C | NPA3 | NP_009197 | XAB 1 | 56.06 | |
| YER126C | NSA2 | NP_055701 | TINP1 | 62.07 | |
| YJL041W | NSP1 | NP_714941 | NUP62 | 26.82 | |
| YER009W | NTF2 | NP_005787 | NUTF2 | 45.61 | |
| YOR373W | NUD1 | NP_653249 | FLJ32786 | 29.59 | Dosage |
| | | | | | Rescue |
| YER006W | NUG1 | NP_061940 | GNL3L | 36.77 | |
| YOR098C | NUP1 | NP_005115 | NUP153 | 26.87 | Synthetic |
| | | | | | Rescue |
| YMR047C | NUP116 | NP_005378 | NUP98 | 24.19 | Dosage |
| | | | | | Rescue |
| YGL092W | NUP145 | NP_005378 | NUP98 | 25.69 | |
| YIL115C | NUP159 | NP_055023 | DSPP | 19.76 | Dosage |
| | | | | | Rescue |
| YGR119C | NUP57 | NP_005378 | NUP98 | 34.8 | |
| YPR168W | NUT2 | NP_115662 | TRG20 | 28.21 | |
| YGL055W | OLE1 | NP_005054 | SCD | 39.34 | |
| YML065W | ORC1 | NP_004144 | ORC1L | 29 | Synthetic |
| | | | | | Rescue |
| YBR060C | ORC2 | NP_006181 | ORC2L | 21.68 | Synthetic |
| | | | | | Rescue |
| YPR162C | ORC4 | NP_002543 | ORC4L | 28.65 | |
| YNL261W | ORC5 | NP_002544 | ORC5L | 22.24 | Dosage |
| | | | | | Rescue |
| YJL002C | OST1 | NP_002941 | RPN1 | 26.98 | Dosage |
| | | | | | Rescue |
| YOR103C | OST2 | NP_001335 | DAD1 | 45.05 | |
| YER165W | PAB1 | NP_002559 | PABPC1 | 59.73 | Synthetic |
| | | | | | Rescue |
| YJL104W | PAM16 | NP_057153 | Magmas | 41.03 | Dosage |
| | | | | | Rescue |
| YLR008C | PAM18 | NP_957711 | DNAJC19 | 46.84 | |
| YIR006C | PAN1 | NP_001972 | EPS15 | 24.81 | Synthetic |
| | | | | | Rescue |
| YKR002W | PAP1 | NP_116021 | PAPOLA | 46.41 | Synthetic |
| | | | | | Rescue |
| YDR228C | PCF11 | NP_056969 | PCF11 | 30.57 | Synthetic |
| | | | | | Rescue |
| YEL058W | PCM1 | NP_056414 | PGM3 | 45.33 | |
| YDR081C | PDC2 | NP_663772 | TIGD4 | 23.64 | Synthetic |
| | | | | | Rescue |
| YCL043C | PDI1 | NP_004902 | PDIA4 | 32.29 | Dosage |
| | | | | | Rescue |
| YMR076C | PDS5 | NP_055847 | APRIN | 19.68 | Dosage |
| | | | | | Rescue |
| YBL030C | PET9 | NP_001143 | SLC25A5 | 54.61 | Dosage |
| | | | | | Rescue |
| YNL317W | PFS2 | NP_060853 | WDR33 | 39.34 | |
| YOR122C | PFY1 | NP_955378 | PFN4 | 30.43 | Dosage |
| | | | | | Rescue |
| YBR196C | PGI1 | NP_000166 | GPI | 57.92 | Dosage |
| | | | | | Rescue |
| YCR012W | PGK1 | NP_000282 | PGK1 | 65.78 | |
| YJL097W | PHS1 | NP_940684 | PTPLB | 32.57 | |
| YNL267W | PIK1 | NP_002642 | PIK4CB | 44.33 | Dosage |
| | | | | | Rescue |
| YPR113W | PIS1 | NP_006310 | CDIPT | 40.4 | |
| YBL105C | PKC1 | NP_006247 | PKN2 | 50 | Synthetic |
| | | | | | Rescue |
| YOR281C | PLP2 | NP_689614 | PDCL2 | 32.57 | |
| YGL008C | PMA1 | NP_055676 | KIAA0703 | 27.45 | Dosage |
| | | | | | Rescue |
| YER003C | PMI40 | NP_002426 | MPI | 39.63 | |
| YML069W | POB3 | NP_003137 | SSRP1 | 31.27 | Synthetic |
| | | | | | Rescue |
| YNL102W | POL1 | NP_058633 | POLA | 35.34 | Dosage |
| | | | | | Rescue |
| YBL035C | POL12 | NP_002680 | POLA2 | 27.92 | |
| YNL262W | POL2 | NP_006222 | POLE | 43.24 | Dosage |
| | | | | | Rescue |
| YBR088C | POL30 | NP_002583 | PCNA | 35.52 | Synthetic |
| | | | | | Rescue |
| YBR257W | POP4 | NP_006618 | POP4 | 33.81 | |
| YER012W | PRE1 | NP_002785 | PSMB2 | 44.85 | Dosage |
| | | | | | Rescue |
| YOR362C | PRE10 | NP_002779 | PSMA3 | 51.61 | |
| YPR103W | PRE2 | NP_002788 | PSMB5 | 66.83 | Synthetic |
| | | | | | Rescue |
| YJL001W | PRE3 | NP_002789 | PSMB6 | 56.06 | |
| YFR050C | PRE4 | NP_002787 | PSMB4 | 43.1 | Dosage |
| | | | | | Rescue |
| YMR314W | PRE5 | NP_002777 | PSMA1 | 52.77 | |
| YOL038W | PRE6 | NP_002783 | PSMA7 | 58.8 | |
| YBL041W | PRE7 | NP_002784 | PSMB1 | 45.65 | |
| YML092C | PRE8 | NP_002778 | PSMA2 | 57.32 | Dosage |
| | | | | | Rescue |
| YIR008C | PRI1 | NP_000937 | PRIM1 | 31.07 | |
| YKL045W | PRI2 | NP_000938 | PRIM2A | 35.95 | |
| YER023W | PRO3 | NP_075566 | PYCRL | 34.74 | |
| YDL043C | PRP11 | NP_009096 | SF3A2 | 27.94 | Dosage |
| | | | | | Rescue |
| YKR086W | PRP16 | NP_054722 | DHX38 | 50.87 | Synthetic |
| | | | | | Rescue |
| YLL036C | PRP19 | NP_055317 | PRPF19 | 38.19 | |
| YNR011C | PRP2 | NP_003578 | DHX16 | 46.79 | Dosage |
| | | | | | Rescue |
| YJL203W | PRP21 | NP_005868 | SF3A1 | 22.88 | |
| YER013W | PRP22 | NP_004932 | DHX8 | 49.75 | Synthetic |
| | | | | | Rescue |
| YMR268C | PRP24 | NP_003810 | PABPC4 | 21.55 | Synthetic |
| | | | | | Rescue |
| YDR243C | PRP28 | NP_004809 | DDX23 | 37.22 | Synthetic |
| | | | | | Rescue |
| YDR473C | PRP3 | NP_004689 | PRPF3 | 28.94 | Synthetic |
| | | | | | Rescue |
| YGR091W | PRP31 | NP_056444 | PRPF31 | 31.07 | |
| YML046W | PRP39 | NP_060392 | PRPF39 | 25 | |
| YPR178W | PRP4 | NP_004688 | PRPF4 | 33.41 | Synthetic |
| | | | | | Rescue |
| YKL012W | PRP40 | NP_001026868 | HYPC | 23.26 | |
| YGL120C | PRP43 | NP_001349 | DHX15 | 65.69 | |
| YAL032C | PRP45 | NP_036377 | SNW1 | 26.61 | |
| YPL151C | PRP46 | NP_002660 | PLRG1 | 50.15 | |
| YBR237W | PRP5 | NP_055644 | DDX46 | 37.64 | Dosage |
| | | | | | Rescue |
| YBR055C | PRP6 | NP_036601 | C20orf14 | 26.76 | |
| YDL030W | PRP9 | NP_006793 | SF3A3 | 26.22 | Synthetic |
| | | | | | Rescue |
| YOR361C | PRT1 | NP_003742 | EIF3S9 | 31.46 | Synthetic |
| | | | | | Rescue |
| YDL055C | PSA1 | NP_068806 | GMPPB | 57.89 | Dosage |
| | | | | | Rescue |
| YMR308C | PSE1 | NP_002262 | RANBP5 | 29.67 | |
| YDR013W | PSF1 | NP_066545 | PSF1 | 28.42 | Dosage |
| | | | | | Rescue |
| YJL072C | PSF2 | NP_057179 | Pfs2 | 26.06 | |
| YOR157C | PUP1 | NP_002790 | PSMB7 | 54.96 | |
| YGR253C | PUP2 | NP_002781 | PSMA5 | 62.34 | |
| YER094C | PUP3 | NP_002786 | PSMB3 | 54.73 | |
| YLR196W | PWP1 | NP_008993 | PWP1 | 33.02 | |
| YCR057C | PWP2 | NP_005040 | PWP2H | 43.6 | |
| YGR280C | PXR1 | NP_060354 | PINX1 | 37.5 | |
| YPR186C | PZF1 | NP_940928 | ZNF710 | 33.46 | |
| YHR074W | QNS1 | NP_060631 | NADSYN1 | 58.59 | |
| YDL103C | QRI1 | NP_003106 | UAP1 | 39.92 | |
| YER171W | RAD3 | NP_000391 | ERCC2 | 52.45 | Dosage |
| | | | | | Rescue |
| YPL153C | RAD53 | NP_065172 | CAMK1G | 43.51 | Synthetic |
| | | | | | Rescue |
| YKL019W | RAM2 | NP_002018 | FNTA | 26.75 | |
| YOR048C | RAT1 | NP_036387 | XRN2 | 44.58 | |
| YDR527W | RBA50 | NP_056355 | RPAP1 | 35.48 | |
| YOL010W | RCL1 | NP_005763 | RCL1 | 36.76 | |
| YPR094W | RDS3 | NP_116147 | PHF5A | 56.19 | |
| YBR002C | RER2 | NP_995583 | DHDDS | 43.59 | Dosage |
| | | | | | Rescue |
| YOR207C | RET1 | NP_060552 | POLR3B | 62.98 | |
| YFR051C | RET2 | NP_001646 | ARCN1 | 43.45 | |
| YPL010W | RET3 | NP_057141 | COPZ1 | 40.94 | Dosage |
| | | | | | Rescue |
| YAR007C | RFA1 | NP_002936 | RPA1 | 31.62 | Dosage |
| | | | | | Rescue |
| YNL312W | RFA2 | NP_002937 | RPA2 | 26.86 | |
| YOR217W | RFC1 | NP_002904 | RFC1 | 35 | Synthetic |
| | | | | | Rescue |
| YJR068W | RFC2 | NP_853551 | RFC4 | 52.6 | Dosage |
| | | | | | Rescue |
| YNL290W | RFC3 | NP_031396 | RFC5 | 50 | |
| YOL094C | RFC4 | NP_852136 | RFC2 | 61.22 | Dosage |
| | | | | | Rescue |
| YBR087W | RFC5 | NP_002906 | RFC3 | 44 | Dosage |
| | | | | | Rescue |
| YBL020W | RFT1 | NP_443091 | RFT1 | 26.1 | |
| YPR165W | RH01 | NP_001655 | RHOA | 75.14 | Dosage |
| | | | | | Rescue |
| YIL118W | RHO3 | NP_001655 | RHOA | 50.25 | Dosage |
| | | | | | Rescue |
| YNL163C | RIA1 | NP_078856 | EFTUD1 | 46.68 | Dosage |
| | | | | | Rescue |
| YOL066C | RIB2 | NP_689473 | RPUSD2 | 50.18 | |
| YBR192W | RIM2 | NP_060625 | SLC25A36 | 39.63 | |
| YOR119C | RIO1 | NP_113668 | RIOK1 | 41.3 | |
| YNL207W | RI02 | NP_060813 | RIOK2 | 45.24 | |
| YLL034C | RIX7 | NP_996671 | NVL | 49.6 | |
| YOR095C | RKI1 | NP_653164 | RPIA | 45.83 | |
| YDR091C | RLI1 | NP_002931 | ABCE1 | 67.6 | |
| YLR009W | RLP24 | NP_057388 | C15orf15 | 60.74 | Dosage |
| | | | | | Rescue |
| YNL002C | RLP7 | XP_371757 | LOC389305 | 38.79 | |
| YMR235C | RNA1 | NP_002874 | RANGAP1 | 30.77 | Synthetic |
| | | | | | Rescue |
| YMR061W | RNA14 | NP_001317 | CSTF3 | 26.73 | Synthetic |
| | | | | | Rescue |
| YGL044C | RNA15 | NP_001316 | CSTF2 | 47.95 | Synthetic |
| | | | | | Rescue |
| YJL026W | RNR2 | NP_001025 | RRM2 | 69.52 | Dosage |
| | | | | | Rescue |
| YGL171W | ROK1 | NP_008941 | DDX52 | 39.38 | |
| YPR010C | RPA135 | NP_061887 | POLR1B | 43.59 | |
| YOR341W | RPA190 | NP_056240 | POLR1A | 36 | Synthetic |
| | | | | | Rescue |
| YOR210W | RPB10 | NP_066951 | POLR2L | 73.53 | Dosage |
| | | | | | Rescue |
| YOL005C | RPB11 | NP_006225 | POLR2J | 52.38 | |
| YOR151C | RPB2 | NP_000929 | POLR2B | 58.15 | |
| YIL021W | RPB3 | NP_002685 | POLR2C | 45.56 | |
| YBR154C | RPB5 | NP_002686 | POLR2E | 43.78 | |
| YDR404C | RPB7 | NP_002687 | POLR2G | 42.77 | |
| YOR224C | RPB8 | NP_006223 | POLR2H | 35.06 | Dosage |
| | | | | | Rescue |
| YDR045C | RPC11 | NP_057394 | POLR3K | 52.21 | |
| YNL113W | RPC19 | NP_057056 | POLR1 D | 45.38 | Dosage |
| | | | | | Rescue |
| YKL144C | RPC25 | NP_001018060 | POLR3H | 42.99 | Dosage |
| | | | | | Rescue |
| YNR003C | RPC34 | NP_006457 | POLR3F | 30.04 | |
| YPR110C | RPC40 | NP_976035 | POLR1 C | 46.67 | Dosage |
| | | | | | Rescue |
| YPR190C | RPC82 | NP_006459 | POLR3C | 22.7 | Dosage |
| | | | | | Rescue |
| YHR088W | RPF1 | NP_079341 | BXDC5 | 46.49 | Dosage |
| | | | | | Rescue |
| YKR081C | RPF2 | NP_115570 | BXDC1 | 46.26 | Dosage |
| | | | | | Rescue |
| YBR079C | RPG1 | NP_003741 | EIF3S10 | 27.18 | Dosage |
| | | | | | Rescue |
| YLR075W | RPL10 | NP_006004 | RPL10 | 67.15 | Dosage |
| | | | | | Rescue |
| YLR029C | RPL15A | NP_002939 | RPL15 | 71.57 | |
| YKL180W | RPL17A | NP_001030178 | RPL17 | 58.78 | |
| YOL120C | RPL18A | NP_000970 | RPL18 | 54.78 | |
| YOL127W | RPL25 | NP_000975 | RPL23A | 65.25 | |
| YGL103W | RPL28 | NP_000981 | RPL27A | 61.74 | |
| YFR031C-A | RPL2A | NP_150644 | RPL8 | 71.65 | |
| YOR063W | RPL3 | NP_000958 | RPL3 | 66.67 | |
| YGL030W | RPL30 | NP_000980 | RPL30 | 61 | |
| YBL092W | RPL32 | NP_001007075 | RPL32 | 62.7 | |
| YPL143W | RPL33A | NP_000987 | RPL35A | 50.46 | |
| YNL162W | RPL42A | NP_066357 | RPL36A | 73.15 | |
| YPL131W | RPL5 | NP_000960 | RPL5 | 50 | |
| YFR004W | RPN11 | NP_005796 | PSMD14 | 68.42 | |
| YFR052W | RPN12 | NP_002803 | PSMD8 | 34.09 | Dosage |
| | | | | | Rescue |
| YIL075C | RPN2 | NP_002798 | PSMD1 | 40.14 | Dosage |
| | | | | | Rescue |
| YER021W | RPN3 | NP_002800 | PSMD3 | 32.42 | Dosage |
| | | | | | Rescue |
| YDL147W | RPN5 | NP_002807 | PSMD12 | 41.12 | |
| YDL097C | RPN6 | NP_002806 | PSMD11 | 45.93 | |
| YPR108W | RPN7 | NP_055629 | PSMD6 | 36.88 | |
| YOR261C | RPN8 | NP_002802 | PSMD7 | 50.33 | |
| YDR427W | RPN9 | NP_002808 | PSMD13 | 31.3 | |
| YDL140C | RPO21 | NP_000928 | POLR2A | 52.3 | Synthetic |
| | | | | | Rescue |
| YPR187W | RPO26 | NP_068809 | POLR2F | 50 | Synthetic |
| | | | | | Rescue |
| YOR116C | RPO31 | NP_008986 | POLR3A | 50.73 | Dosage |
| | | | | | Rescue |
| YLR340W | RPP0 | NP_000993 | RPLP0 | 55.19 | |
| YHR062C | RPP1 | NP_006404 | RPP30 | 29.2 | |
| YDR064W | RPS13 | NP_001008 | RPS13 | 78 | |
| YOL040C | RPS15 | NP_001009 | RPS15 | 59.31 | |
| YGL123W | RPS2 | NP_002943 | RPS2 | 61.57 | |
| YHL015W | RPS20 | NP_001014 | RPS20 | 62.89 | |
| YNL178W | RPS3 | NP_000996 | RPS3 | 65.42 | |
| YLR167W | RPS31 | NP_002945 | RPS27A | 82.78 | |
| YJR123W | RPS5 | NP_001000 | RPS5 | 71.96 | Dosage |
| | | | | | Rescue |
| YKL145W | RPT1 | NP_002794 | PSMC2 | 72.67 | Synthetic |
| | | | | | Rescue |
| YDL007W | RPT2 | NP_002793 | PSMC1 | 73.32 | |
| YDR394W | RPT3 | NP_006494 | PSMC4 | 66.82 | |
| YOR259C | RPT4 | NP_002797 | PSMC6 | 68.05 | |
| YOR117W | RPT5 | NP_002795 | PSMC3 | 69.45 | |
| YGL048C | RPT6 | NP_002796 | PSMC5 | 76.61 | Synthetic |
| | | | | | Rescue |
| YMR131C | RRB1 | NP_113673 | GRWD1 | 33.92 | |
| YKL125W | RRN3 | NP_060897 | RRN3 | 25.16 | |
| YDR087C | RRP1 | NP_055871 | KIAA0179 | 36.36 | Synthetic |
| | | | | | Rescue |
| YPL012W | RRP12 | NP_055994 | KIAA0690 | 24.33 | |
| YHR065C | RRP3 | NP_057439 | DDX47 | 56.68 | |
| YHR069C | RRP4 | NP_055100 | EXOSC2 | 41.55 | |
| YDL111C | RRP42 | NP_055819 | EXOSC7 | 25.1 | |
| YDR280W | RRP45 | NP_005024 | EXOSC9 | 34.83 | |
| YGR095C | RRP46 | NP_064543 | EXOSC5 | 28.17 | |
| YMR229C | RRP5 | NP_055791 | PDCD11 | 25.32 | Dosage |
| | | | | | Rescue |
| YPR137W | RRP9 | NP_004695 | RNU3IP2 | 26.44 | |
| YOR294W | RRS1 | NP_055984 | RRS1 | 35.05 | |
| YCR072C | RSA4 | NP_060566 | NLE1 | 46.63 | |
| YKR008W | RSC4 | NP_851385 | PB1 | 27.27 | Synthetic |
| | | | | | Rescue |
| YFR037C | RSC8 | NP_620706 | SMARCC2 | 34.35 | Dosage |
| | | | | | Rescue |
| YML049C | RSE1 | NP_036558 | SF3B3 | 23.92 | Dosage |
| | | | | | Rescue |
| YER125W | RSP5 | NP_056092 | NEDD4L | 49.25 | Dosage |
| | | | | | Rescue |
| YOR077W | RTS2 | NP_036443 | KIN | 34.21 | |
| YDR190C | RVB1 | NP_003698 | RUVBL1 | 70.74 | |
| YPL235W | RVB2 | NP_006657 | RUVBL2 | 68.76 | |
| YFR005C | SAD1 | NP_006581 | USP39 | 29.91 | Synthetic |
| | | | | | Rescue |
| YER043C | SAH1 | NP_000678 | AHCY | 71.04 | |
| YNL026W | SAM50 | NP_056195 | SAMM50 | 32.3 | |
| YPL218W | SAR1 | NP_001028675 | SAR1B | 60 | Dosage |
| | | | | | Rescue |
| YDR180W | SCC2 | NP_597677 | NIPBL | 19.82 | |
| YGL011C | SCL1 | NP_002782 | PSMA6 | 54.32 | |
| YGR245C | SDA1 | NP_060585 | SDAD1 | 31 | Dosage |
| | | | | | Rescue |
| YKL193C | SDS22 | NP_002703 | PPP1R7 | 46.36 | Dosage |
| | | | | | Rescue |
| YDR164C | SEC1 | NP_008880 | STXBP2 | 25.7 | Dosage |
| | | | | | Rescue |
| YLR166C | SEC10 | NP_006535 | EXOC5 | 24.43 | Dosage |
| | | | | | Rescue |
| YIR022W | SEC11 | NP_055115 | SEC11L1 | 49.67 | Dosage |
| | | | | | Rescue |
| YLR208W | SEC13 | NP_899195 | SEC13L1 | 50.83 | Dosage |
| | | | | | Rescue |
| YMR079W | SEC14 | XP_032693 | KIAA0420 | 31.14 | Synthetic |
| | | | | | Rescue |
| YGL233W | SEC15 | XP_039570 | SEC15L2 | 20.25 | Dosage |
| | | | | | Rescue |
| YPL085W | SEC16 | NP_055023 | DSPP | 17.23 | Dosage |
| | | | | | Rescue |
| YBL050W | SEC17 | NP_003818 | NAPA | 34.71 | Dosage |
| | | | | | Rescue |
| YBR080C | SEC18 | NP_006169 | NSF | 45.91 | |
| YNL287W | SEC21 | NP_057212 | COPG | 34.26 | Synthetic |
| | | | | | Rescue |
| YPR181C | SEC23 | NP_116781 | SEC23B | 50.07 | Dosage |
| | | | | | Rescue |
| YIL109C | SEC24 | NP_006314 | SEC24B | 33.51 | Dosage |
| | | | | | Rescue |
| YDR238C | SEC26 | NP_057535 | COPB | 42.93 | |
| YGL137W | SEC27 | NP_004757 | COPB2 | 47.04 | Dosage |
| | | | | | Rescue |
| YDL195W | SEC31 | NP_057295 | SEC31L1 | 24.43 | Dosage |
| | | | | | Rescue |
| YFL005W | SEC4 | NP_057614 | RAB8B | 61.05 | Dosage |
| | | | | | Rescue |
| YFL045C | SEC53 | NP_000294 | PMM2 | 59.83 | |
| YMR013C | SEC59 | NP_055723 | TMEM15 | 29.32 | |
| YIL068C | SEC6 | NP_009208 | EXOC3 | 22.34 | Synthetic |
| | | | | | Rescue |
| YLR378C | SEC61 | NP_037468 | SEC61A1 | 56.8 | Dosage |
| | | | | | Rescue |
| YPL094C | SEC62 | NP_003253 | TLOC1 | 30.86 | Dosage |
| | | | | | Rescue |
| YOR254C | SEC63 | NP_009145 | SEC63 | 26.65 | Dosage |
| | | | | | Rescue |
| YDR170C | SEC7 | NP_006412 | ARFGEF1 | 31.29 | Dosage |
| | | | | | Rescue |
| YPR055W | SEC8 | NP_068579 | EXOC4 | 17.97 | Dosage |
| | | | | | Rescue |
| YLR026C | SED5 | NP_003155 | STX5A | 30.67 | Dosage |
| | | | | | Rescue |
| YLR430W | SEN1 | NP_055861 | ALS4 | 29 | |
| YLR105C | SEN2 | NP_079541 | TSEN2 | 30.41 | |
| YDR023W | SES1 | NP_006504 | SARS | 44.99 | |
| YLR321C | SFH1 | NP_003064 | SMARCB1 | 30.73 | Dosage |
| | | | | | Rescue |
| YLR336C | SGD1 | NP_612409 | NOM1 | 31.69 | |
| YOR057W | SGT1 | NP_006695 | SUGT1 | 33.18 | Dosage |
| | | | | | Rescue |
| YPR161C | SGV1 | NP_277074 | CDC2L2 | 34.99 | Synthetic |
| | | | | | Rescue |
| YIL104C | SHQ1 | NP_060600 | SHQ1 | 25.87 | |
| YLR197W | SIK1 | NP_006383 | NOL5A | 54.21 | |
| YNL007C | SIS1 | NP_008965 | DNAJB4 | 39.88 | Synthetic |
| | | | | | Rescue |
| YGR195W | SK16 | NP_061910 | EXOSC4 | 35.42 | |
| YDR328C | SKP1 | NP_733779 | SKP1A | 42.93 | Dosage |
| | | | | | Rescue |
| YDR489W | SLD5 | NP_115712 | SLD5 | 22.18 | Dosage |
| | | | | | Rescue |
| YDR088C | SLU7 | NP_006416 | SLU7 | 23.1 | Dosage |
| | | | | | Rescue |
| YDR189W | SLY1 | NP_057190 | SCFD1 | 32.8 | Dosage |
| | | | | | Rescue |
| YFL008W | SMC1 | NP_006297 | SMC1L1 | 29.87 | Dosage |
| | | | | | Rescue |
| YFR031C | SMC2 | NP_006435 | SMC2L1 | 36.97 | Dosage |
| | | | | | Rescue |
| YJL074C | SMC3 | NP_005436 | CSPG6 | 31.99 | |
| YLR086W | SMC4 | NP_005487 | SMC4L1 | 36.86 | |
| YOL034W | SMC5 | NP_055925 | SMC5L1 | 26.72 | Synthetic |
| | | | | | Rescue |
| YGR074W | SMD1 | NP_008869 | SNRPD1 | 39.04 | |
| YLR275W | SMD2 | NP_808210 | SNRPD2 | 52.68 | |
| YLR147C | SMD3 | NP_004166 | SNRPD3 | 47.67 | |
| YOR159C | SME1 | NP_003085 | SNRPE | 49.41 | |
| YOR149C | SMP3 | NP_079439 | SMP3 | 29.68 | |
| YDR510W | SMT3 | NP_003343 | SUMO1 | 48.42 | Dosage |
| | | | | | Rescue |
| YFL017W-A | SMX2 | NP_003087 | SNRPG | 54.79 | |
| YPR182W | SMX3 | NP_003086 | SNRPF | 59.15 | |
| YIL061C | SNP1 | NP_003080 | SNRP70 | 31.98 | Dosage |
| | | | | | Rescue |
| YKL173W | SNU114 | NP_004238 | EFTUD2 | 33.9 | |
| YEL026W | SNU13 | NP_001003796 | NHP2L1 | 71.31 | |
| YLL011W | SOF1 | NP_056235 | WDSOF1 | 45.61 | |
| YOR353C | SOG2 | NP_694992 | LRRC57 | 31.65 | Dosage |
| | | | | | Rescue |
| YCL054W | SPB1 | NP_060117 | FTSJ3 | 40.68 | |
| YFL002C | SPB4 | NP_065987 | DDX55 | 39.41 | |
| YDR356W | SPC110 | NP_829884 | RAB6IP2 | 21.52 | Dosage |
| | | | | | Rescue |
| YLR066W | SPC3 | NP_068747 | SPCS3 | 36.96 | |
| YHR172W | SPC97 | NP_006650 | TUBGCP2 | 20.54 | Dosage |
| | | | | | Rescue |
| YNL126W | SPC98 | NP_006313 | TUBGCP3 | 24.88 | Dosage |
| | | | | | Rescue |
| YPL175W | SPT14 | NP_002632 | PIGA | 45.37 | |
| YER148W | SPT15 | NP_003185 | TBP | 77.32 | Synthetic |
| | | | | | Rescue |
| YGL207W | SPT16 | NP_009123 | SUPT16H | 34.26 | Dosage |
| | | | | | Rescue |
| YML010W | SPT5 | NP_003160 | SUPT5H | 29.25 | Synthetic |
| | | | | | Rescue |
| YGR116W | SPT6 | NP_003161 | SUPT6H | 23.64 | Synthetic |
| | | | | | Rescue |
| YIR012W | SQT1 | NP_001078 | AAMP | 22.19 | |
| YGL097W | SRM1 | NP_001260 | RCC1 | 26.87 | Synthetic |
| | | | | | Rescue |
| YNL189W | SRP1 | NP_036448 | KPNA6 | 53.32 | Dosage |
| | | | | | Rescue |
| YDR292C | SRP101 | NP_003130 | SRPR | 40.3 | |
| YKL154W | SRP102 | NP_067026 | SRPRB | 25.7 | Dosage |
| | | | | | Rescue |
| YPR088C | SRP54 | NP_003127 | SRP54 | 53.72 | |
| YPL243W | SRP68 | NP_055045 | SRP68 | 21.68 | |
| YJR045C | SSC1 | NP_004125 | HSPA9B | 66.56 | Dosage |
| | | | | | Rescue |
| YLR005W | SSL1 | NP_001506 | GTF2H2 | 39.09 | Synthetic |
| | | | | | Rescue |
| YIL143C | SSL2 | NP_000113 | ERCC3 | 55.54 | |
| YNL222W | SSU72 | NP_054907 | SSU72 | 45.55 | Synthetic |
| | | | | | Rescue |
| YIL126W | STH1 | NP_620614 | SMARCA2 | 53.22 | Dosage |
| | | | | | Rescue |
| YGL022W | STT3 | NP_849193 | SIMP | 53.87 | Dosage |
| | | | | | Rescue |
| YLR305C | STT4 | NP_477352 | PIK4CA | 28.18 | Dosage |
| | | | | | Rescue |
| YLR045C | STU2 | NP_055571 | CKAP5 | 24.22 | Synthetic |
| | | | | | Rescue |
| YPR086W | SUA7 | NP_001505 | GTF2B | 35.35 | Dosage |
| | | | | | Rescue |
| YDL084W | SUB2 | NP_542165 | BAT1 | 62.87 | Dosage |
| | | | | | Rescue |
| YNL244C | SUI1 | NP_005866 | GC20 | 56.76 | Synthetic |
| | | | | | Rescue |
| YJR007W | SUI2 | NP_004085 | EIF2S1 | 54.27 | Dosage |
| | | | | | Rescue |
| YPL237W | SUI3 | NP_003899 | EIF2S2 | 47.77 | |
| YDR172W | SUP35 | NP_002085 | GSPT1 | 57.51 | Dosage |
| | | | | | Rescue |
| YBR143C | SUP45 | NP_004721 | ETF1 | 69.9 | Dosage |
| | | | | | Rescue |
| YGR002C | SWC4 | NP_001029196 | DMAP1 | 22.41 | |
| YKL018W | SWD2 | XP_293514 | LOC344620 | 33.33 | Dosage |
| | | | | | Rescue |
| YDR416W | SYF1 | NP_064581 | XAB2 | 27.14 | |
| YJL035C | TAD2 | NP_872309 | DEADC1 | 40 | |
| YLR316C | TAD3 | NP_612431 | LOC113179 | 26.07 | |
| YGR274C | TAF1 | NP_620278 | TAF1 | 28.88 | Dosage |
| | | | | | Rescue |
| YDR167W | TAF10 | NP_006275 | TAF10 | 30.3 | Dosage |
| | | | | | Rescue |
| YDR145W | TAF12 | NP_005635 | TAF12 | 38.46 | |
| YML098W | TAF13 | NP_005636 | TAF13 | 30.3 | |
| YBR198C | TAF5 | NP_008882 | TAF5 | 32.71 | |
| YGL112C | TAF6 | NP_647476 | TAF6 | 31.42 | Dosage |
| | | | | | Rescue |
| YMR227C | TAF7 | NP_079161 | TAF7L | 29.17 | |
| YMR236W | TAF9 | NP_003178 | TAF9 | 32.84 | Dosage |
| | | | | | Rescue |
| YPR048W | TAH18 | NP_055249 | NDOR1 | 29.4 | |
| YIL129C | TAO3 | XP_093839 | KIAA0826 | 21.09 | Synthetic |
| | | | | | Rescue |
| YMR028W | TAP42 | NP_001542 | IGBP1 | 23.87 | Dosage |
| | | | | | Rescue |
| YDR212W | TCP1 | NP_110379 | TCP1 | 64.27 | |
| YML064C | TEM1 | NP_065724 | RAB22A | 29.38 | Dosage |
| | | | | | Rescue |
| YKL028W | TFA1 | NP_005504 | GTF2E1 | 28.44 | Dosage |
| | | | | | Rescue |
| YDR311W | TFB1 | NP_005307 | GTF2H1 | 35.71 | |
| YPL122C | TFB2 | NP_001508 | GTF2H4 | 34.32 | |
| YDR460W | TFB3 | NP_002422 | MNAT1 | 43.24 | |
| YPR056W | TFB4 | NP_001507 | GTF2H3 | 30.53 | |
| YGR047C | TFC4 | NP_036218 | GTF3C3 | 23.93 | Dosage |
| | | | | | Rescue |
| YGR005C | TFG2 | NP_004119 | GTF2F2 | 26.25 | |
| YGR024C | THG1 | NP_060342 | ICF45 | 52.56 | |
| YOR143C | THI80 | NP_071890 | TPK1 | 28.89 | |
| YIL078W | THS1 | NP_689508 | TARS | 56.81 | |
| YIL144W | TID3 | NP_006092 | KNTC2 | 20.92 | Dosage |
| | | | | | Rescue |
| YMR260C | TIF11 | NP_001403 | EIF1AX | 65.22 | |
| YMR146C | TIF34 | NP_003748 | EIF3S2 | 44.88 | Dosage |
| | | | | | Rescue |
| YDR429C | TIF35 | NP_003746 | EIF3S4 | 31.75 | |
| YPR041W | TIF5 | NP_892116 | EIF5 | 37.16 | |
| YPR016C | TIF6 | NP_002203 | ITGB4BP | 72.84 | |
| YJL143W | TIM17 | NP_005825 | TIMM17B | 49.35 | Dosage |
| | | | | | Rescue |
| YDL217C | TIM22 | NP_037469 | TIMM22 | 34.97 | |
| YIL022W | TIM44 | NP 006342 | TIMM44 | 26.89 | Dosage |
| | | | | | Rescue |
| YPL063W | TIM50 | NP_001001563 | TIMM50 | 37.21 | |
| YOR194C | TOA1 | NP_056943 | GTF2A1 | 43.75 | |
| YKL058W | TOA2 | NP_004483 | GTF2A2 | 37.39 | Dosage |
| | | | | | Rescue |
| YMR203W | TOM40 | NP_006105 | TOMM40 | 25 | Dosage |
| | | | | | Rescue |
| YNL088W | TOP2 | NP_001058 | TOP2A | 45.4 | |
| YKL203C | TOR2 | NP_004949 | FRAP1 | 43.48 | Dosage |
| | | | | | Rescue |
| YDR050C | TP11 | NP_000356 | TP11 | 53.25 | |
| YOL102C | TPT1 | NP_001028850 | TRPT1 | 39.36 | |
| YNR046W | TRM112 | NP_057488 | HSPC152 | 30 | |
| YHR070W | TRM5 | NP_065861 | TRMT5 | 36.33 | |
| YBR254C | TRS20 | NP_001011658 | TRAPPC2 | 33.53 | |
| YDR246W | TRS23 | NP_057230 | TRAPPC4 | 28.02 | |
| YDR472W | TRS31 | XP_058961 | TRAPPC5 | 31.3 | |
| YKR079C | TRZ1 | NP_060597 | ELAC2 | 25.09 | |
| YBR265W | TSC10 | NP_002026 | FVT1 | 24.52 | |
| YER093C | TSC11 | XP_380173 | MGC39830 | 25.76 | Dosage |
| | | | | | Rescue |
| YDL015C | TSC13 | NP_612510 | GPSN2 | 34.65 | |
| YDL060W | TSR1 | NP_060598 | FLJ10534 | 29.29 | |
| YML085C | TUB1 | NP_005991 | TUBA1 | 74.44 | Dosage |
| | | | | | Rescue |
| YFL037W | TUB2 | NP_006078 | TUBB4 | 77.39 | Synthetic |
| | | | | | Rescue |
| YLR212C | TUB4 | NP_057521 | TUBG2 | 40.53 | Dosage |
| | | | | | Rescue |
| YGR185C | TYS1 | NP_003671 | YARS | 56.37 | |
| YKL210W | UBA1 | NP_003325 | UBE1 | 52.36 | |
| YDR390C | UBA2 | NP_005490 | UBA2 | 34.01 | |
| YDR177W | UBC1 | NP_005330 | HIP2 | 43.01 | |
| YDL064W | UBC9 | NP_919237 | UBE21 | 56.41 | Dosage |
| | | | | | Rescue |
| YGR048W | UFD1 | NP_005650 | UFD1L | 38.12 | |
| YKL035W | UGP1 | NP_001001521 | UGP2 | 55.7 | |
| YPL020C | ULP1 | NP_055369 | SENP1 | 32.21 | Synthetic |
| | | | | | Rescue |
| YIL031W | ULP2 | NP_065705 | SENP7 | 24.32 | Synthetic |
| | | | | | Rescue |
| YKL024C | URA6 | NP_057392 | CMPK | 50.54 | |
| YDL058W | USO1 | NP_003706 | VDP | 22.05 | Dosage |
| | | | | | Rescue |
| YJL109C | UTP10 | XP_375853 | FLJ10359 | 23.25 | |
| YKL099C | UTP11 | NP_057121 | UTP11L | 30.43 | |
| YLR222C | UTP13 | NP_006444 | TBL3 | 30.18 | |
| YML093W | UTP14 | NP_067677 | UTP14C | 27.99 | |
| YMR093W | UTP15 | NP_115551 | UTP15 | 31.21 | |
| YJL069C | UTP18 | NP_057085 | WDR50 | 25.54 | |
| YBL004W | UTP20 | NP_055318 | DRIM | 23.06 | |
| YLR409C | UTP21 | NP_644810 | WDR36 | 31.88 | |
| YGR090W | UTP22 | NP_075068 | NOL6 | 23.52 | |
| YDR324C | UTP4 | NP_116219 | CIRH1A | 23.56 | |
| YDR449C | UTP6 | NP_060898 | C17orf40 | 32.28 | |
| YER082C | UTP7 | NP_005443 | WDR46 | 40.99 | |
| YGR094W | VAS1 | NP_006286 | VARS | 50.15 | |
| YMR197C | VTI1 | NP_660207 | VTI1A | 32.81 | Dosage |
| | | | | | Rescue |
| YEL002C | WBP1 | NP_005207 | DDOST | 25.06 | Dosage |
| | | | | | Rescue |
| YOL097C | WRS1 | NP_004175 | WARS | 54.8 | |
| YPL252C | YAH1 | NP_001026904 | MGC19604 | 45.45 | |
| YDR325W | YCG1 | NP_071741 | HCAP-G | 22.59 | |
| YLR272C | YCS4 | NP_055680 | CNAP1 | 25.54 | |
| YDL193W | YDL193W | XP_372205 | LOC389850 | 27.54 | |
| YDR196C | YDR196C | NP_079095 | FLJ22955 | 37.87 | |
| YDR267C | YDR267C | NP_004795 | WDR39 | 42.77 | |
| YDR339C | YDR339C | NP_057046 | C14orf111 | 61.98 | |
| YDR341C | YDR341C | NP_064716 | RARSL | 41.56 | |
| YDR531 W | YDR531 W | NP_060686 | PANK4 | 38.44 | |
| YLR249W | YEF3 | NP_001020262 | ABCF1 | 29.93 | Dosage |
| | | | | | Rescue |
| YER036C | YER036C | NP_009120 | ABCF2 | 54.04 | |
| YER048W-A | YER048W-A | NP_065141 | C6orf149 | 42.68 | |
| YDL120W | YFH1 | NP_000135 | FXN | 49.3 | Synthetic |
| | | | | | Rescue |
| YGR046W | YGR046W | NP_620162 | MGC16471 | 40.09 | |
| YGR277C | YGR277C | NP_079509 | COASY | 37.5 | |
| YHR020W | YHR020W | NP_004437 | EPRS | 58.1 | |
| YHR122W | YHR122W | NP_057146 | FAM96B | 52.82 | |
| YNL263C | YIF1 | NP_291035 | YIF1B | 28.44 | |
| YIL083C | YIL083C | NP_078940 | PPCS | 40.31 | |
| YIL091C | YIL091C | NP_055203 | C1orf107 | 34.83 | |
| YGR172C | YIP1 | NP_110426 | YIPF5 | 41.44 | Dosage |
| | | | | | Rescue |
| YJL010C | YJL010C | NP_777573 | C14orf21 | 21.58 | |
| YKL095W | YJU2 | NP_060544 | FLJ10374 | 36.63 | |
| YKL033W | YKL033W | NP_055472 | KIAA0406 | 18.31 | |
| YKL033W-A | YKL033W-A | NP_036212 | HDHD1A | 37.23 | |
| YKL088W | YKL088W | NP_068595 | PPCDC | 40.44 | |
| YKL196C | YKT6 | NP_006546 | YKT6 | 47 | Dosage |
| | | | | | Rescue |
| YLR022C | YLR022C | NP_057122 | SBDS | 41.37 | |
| YLR051C | YLR051C | NP_055412 | ERBP | 37.93 | |
| YLR243W | YLR243W | NP_057385 | ATPBD1C | 43.54 | |
| YML125C | YML125C | NP_057327 | CYB5R1 | 36.56 | |
| YNL181W | YNL181W | NP_065956 | RDH14 | 29.64 | |
| YNL247W | YNL247W | NP_001014437 | CARS | 44.61 | |
| YNL313C | YNL313C | NP_060205 | FLJ20272 | 22.92 | |
| YOR004W | YOR004W | NP_115710 | C80rf53 | 31.1 | |
| YOR256C | YOR256C | NP_005458 | NAALAD2 | 23.62 | |
| YOR262W | YOR262W | NP_060536 | ATPBD1B | 47.92 | |
| YOR287C | YOR287C | NP_149103 | C6orf153 | 30.87 | |
| YFL038C | YPT1 | NP_004152 | RAB1A | 70.87 | Dosage |
| | | | | | Rescue |
| YDR002W | YRB1 | NP_002873 | RANBP1 | 56.16 | Dosage |
| | | | | | Rescue |
| YIL063C | YRB2 | NP_003615 | RANBP3 | 30.43 | Dosage |
| | | | | | Rescue |
| YLR277C | YSH1 | NP_057291 | CPSF3 | 40.19 | Synthetic |
| | | | | | Rescue |
| YPR107C | YTH1 | NP_006684 | CPSF4 | 48.23 | |
| YOR272W | YTM1 | NP_060726 | WDR12 | 29.74 | |
| YGR211W | ZPR1 | NP_003895 | ZNF259 | 45.8 | Dosage |
| | | | | | Rescue |

The skilled person will moreover immediately realise that this approach can be used with any non-human organism, for instance, viruses, bacteria, fungi, non-human animals, e.g. mice, fishes, insects or rats, and/or culture of cells, including human cells.

The expression "modulator effect" intends to encompass any effect significantly measurable as compared to a control. Preferred are agonistic or antagonistic effects.

The expression "yeast cell" is used herein to mention unicellular fungi of the phylum Ascomycota that reproduce by fission or budding and are capable of fermenting carbohydrates into alcohol and carbon dioxide. Preferably said yeast cells are Saccharomycetaces or Schizossaccharomycefales, such as Saccharomyces pombe, Saccharomyces cerevisee, Candida or Pichia. Most preferably yeast cells of the species Saccharomyces cervisae are used for manipulation in accordance with the present invention. A genetically stable yeast cell is also referred to as a yeast strain.

A "mutant" refers to an organism which, as a result of either natural processes or mutagenesis techniques including but not limited to site directed mutagenesis, contains one or more mutations (deletions, insertions and/or substitutions) in its genome as compared to the wild-type strain (used for its taxonomic classification), which mutation(s) leads to an inactivation of the target. Preferred embodiments of such mutant according to the present invention are deletion yeast strains, more particularly, deletion strains comprising one or more deletions in one or more genes coding for the target, resulting in the strain lacking a functional gene for the target or a functional target. A functional gene, as used herein refers to a gene which is capable of expressing its natural gene product.

In the context of the present invention, yeast strains "lacking one or more functional genes" are yeast strains in which one or more genes cannot be expressed (no gene product) or which express an aberrant gene product, i.e. a product which is not the natural gene product. This definition also applies *mutatis mutandis* for any non-human organism. Preferably, according to the present invention, such an aberrant gene product, is a gene product which has lost its original function (as present in the wild-type strain). Alternatively such aberrant gene product has a reduced function (less than 50%, less than 75%, or even less than 90%) when compared to the wild type function (enzymatic activity, binding affinity etc.). Yeast cells or strains lacking one or more functional genes can be the result of different factors, such as, but not limited to one or more mutations in the gene itself or in a gene encoding a factor (protein, RNA, dsRNA) which is capable of modifying the expression of said gene, a truncation (caused e.g. by aberrant expression of an enzyme capable of degrading said protein); the complete absence of a gene; the insertion of a transposon; expression of a transgene (resulting in missense, antisense, sense expression) which modifies the expression or the activity of the natural gene product.

The term "compound" is used herein to denote a chemical structure, a mixture of chemical structures, a peptide, a biological substance such as a macromolecule, or an extract made from biological materials such as bacteria, plants, fungi, or animal (particularly mammalian) cells or tissues. Preferred embodiments are chemical structures and a mixture of chemical structures. According to one aspect of the present invention, methods are provided for identifying compounds capable of influencing, modifying (increasing or decreasing) the phenotypic effect of the target gene or protein in yeast. The influence of a compound on the phenotypic effect observed in yeast cells can be direct, by interacting with the target, or can be indirect, by affecting the expression of the target protein in yeast, or by affecting inhibitors or enzymes which themselves influence the phenotypic effect of the target. Alternatively, the effect of these compounds may be by interacting with proteins or enzymes which directly mediate the phenotypic enact of the target. Such compounds include but are not limited to proteins or polypeptides (such as heat shock proteins, receptors, enzymes, ligands, nuclear or cytoplasmic proteins, chaperones, hormones, antigens, growth factors, regulatory proteins, structural proteins) nucleic acids (RNAs, ribozymes, DNAs or cDNAs) or chemical compounds. The screening of compounds from natural or synthetic libraries is also envisaged.

A screening method, as used herein refers to a method for identifying specific compounds. More particularly, the screening method of the present invention allows the identification of compounds based on their ability to specifically modulate the target.

The expressions "heterologuous factor" or "environmental factor" include any factor suitable to compensate for the loss of function of the target in the mutant non-human organism used. Examples of said factors can be temperature, pH, specific down-stream factors such as products of enzymes considered as targets, additional genetic perturbations such as single or multiple gene deletion(s), single or multiple gene overexpression, genes carrying mutations such that their functions are altered. The skilled person is able to provide and or obtain said additional genetic perturbations such as single or multiple gene deletion(s), single or multiple gene overexpression, genes carrying mutations such that their functions are altered by methods which are well-known, for instance the methods described herein-above.

The expression "lipid desaturase" describes any enzyme capable of removing two hydrogen atoms from a lipid molecule to leave a carbon-carbon double bond (e.g. the enzyme HsSCD1 that that converts stearoyl-CoA into oleoyl-CoA).

### Examples

### Material:

### S. cerevisiae strains

(indivudual mutant strains are available from Invitrogen - strains are derived from BY4743). These strains can be constructed using standard yeast techniques, bearing in mind the media requirements for *ole1* deletion mutants i.e. presence of sufficient oleic acid in the media.
*1) a*/*alpha ura3*/*ura3 leu2*/*leu2 his3*/*his3 met15*/*MET15 lys2*/*LYS2 pdr5::kanMX*/ *pdr5::kanMX* (known as *pdr5* / *pdr5* from now on)
*2) a*/*alpha ura3*/*ura3 leu2*/*leu2 his3*/*his3 met15*/*MET15 lys2*/*LYS2 pdr5::kanMX*/ *pdr5::kanMX ole1::kanMX* / *ole1::kanMX* (known as *ole1* / *ole1 pdr5* / *pdr5* from now on)

### Growth medium, buffers

### SD:

SD + Uracil, Leucine, Histidine, 6.7 g/l yeast nitrogen base w/o amino acids (Difco 291940), 2% glucose (w/v) (Difco 0155-17-4). Uracil 20 mg/l (Sigma U-1128), Leucine 100 mg/l (Bio101 4060-522), Histidine 20 mg/l (Sigma H-9511).

### SD + Oleic acid:

SD as above containing 0.1%v/v mixture of NP-40 and oleic acid in the ratio 73:27 - giving a final concentration of 1 mM oleic acid..

### Buffer for Alamar Blue Measurement:

500 mM KPi pH6.4 (184 ml 1M KH₂PO₄ Sigma P-8709, 66 ml 1M K₂HPO₄ Sigma P-8584 to 500 ml with H₂O)

### Alamar Blue solution:

Alamar Blue 100% solution (AlamarBlue^{™} Biosource Europe SA)).

### Plasticware

1536 GNF plates, CatNo: 782076

### Procedure:

### Cell preparation

Yeast cultures were grown overnight in SD (*pdr5*/*pdr5*) or SD + oleic acid (*ole1* / *ole1 pdr5* / *pdr5*)*.* Cells were diluted (1:100) to fresh medium during the day to be seeded for screening in the logarithmic growth phase (between 10⁵ and 10⁶ cells per ml).
Cultures were diluted to concentration of 100'000 cells / ml (*pdr5* / *pdr5*) and 200'000 cells/ml (*ole1* / *ole1 pdr5* / *pdr5*) immediately prior to seeding.

### Dispensing

60 nL of Compound in 90% DMSO were dispensed 1536 well format (60nL) as 3 dilutions per log series - with 16 dilutions in total.

6 µL of Yeast suspension was added to each well of the pre-dispensed compound plates and incubated for 16h *(pdr5*/*pdr5)* or 22h *(ole1*/*ole1 pdr5*/*pdr5)* @ 30C 95% humidity.

After primary incubation 2µL of Alamar Blue solution (3 mL Alamar blue ^{™}, 20 mL 500 mM KPi buffer, pH 6.4) was dispensed to each well and plates were incubated for an additional 3h at 30°C, 95% humidity.

Growth was measured by measuring signal generated by Alamar Blue reduction on a suitable fluorescent plate reader was measured on EnVision (Perkin Elmer) reader (elapsed time 19h for *pdr5*/*pdr5* and 25h for *ole1*/*ole1pdr5*/*pdr5* mutant).

Raw data was normalised and IC50 curves were calculated using spotfire to produce graphs (see figure 2 for examples).

### Conclusion

Several compounds display profiles similar to those in the lower right panel of the above figure. These compounds are considered to have specificity for Ole1 p because they (i) inhibit the growth of yeast expressing Ole1 p, and they (ii) *do not* inhibit yeast cells lacking Ole1 p.

### References

Jiang et al., 2005. "Prevention of obesity in mice by antisense oligonucleotide inhibitors of stearoyl-CoA desaturase-1". J Clin Invest. 2005 Apr;115(4):1030-8 Stukey J., et al. 1990. "The OLE1 gene of Saccharomyces cerevisiae encodes the delta 9 fatty acid desaturase and can be functionally replaced by the rat stearoyl-CoA desaturase gene". J Biol Chem. 1990 Nov 25;265(33):20144-9.

## Claims

1. A screening method for the identification of a target-specific modulator comprising the following steps:
a) screening of a library of potential compounds for modulator effects on a non-human organism comprising the target (wild-type), wherein compounds having modulator effects are identified as potential target-specific modulators, and
b) screening of the potential target-specific modulators identified in step a) for modulator effects on a mutant of the non-human organism of step a), wherein said mutant does not comprise the target or wherein said target is deficient in said mutant, wherein said target is an essential gene and the absence/deficiency of said essential gene in the mutant is specifically compensated by the alteration of other gene(s) (loss and / or gain of function), addition of a heterologuous factor or by an environmental factor; and
wherein a modulator effect in step b) indicates that said potential target-specific modulators is not target-specific whereas the absence of a modulator effect in step b) indicates that said potential target-specific modulators is a specific modulator of said target.

2. The screening method of claim 1 wherein said organism is a yeast or a mouse.

3. The screening method of claims 1 or 2 wherein said target is a homologue of a human target.

4. The screening method of claims 1 to 3 wherein the compound is an inhibitor or an agonist.

5. The method of claims 1 to 4 wherein the non-human organism is a yeast, and growth is assessed in order to evaluate the specific activity of said inhibitor.

6. The method of claims 1 to 5 wherein the essential gene is a metabolic enzyme.

7. The method of claims 1 to 6 wherein the absence/deficiency of said essential gene in the mutant is specifically compensated by the addition of a compensating metabolite.

8. The method of claims 1 to 7 wherein the essential gene is a lipid desaturase and the absence/deficiency of said essential gene in the mutant is specifically compensated by the addition of the desaturated product of said desaturase.

9. The method of claim 1 to 8 wherein the target is ScOle1 p and the absence / deficiency of said essential gene in the mutant is specifically compensated by the addition of oleic acid.
